(19) Europäisches Patentamt
European Patent Office
Office européen des brevets

(11) EP 3 771 927 A1

# (12) DEMANDE DE BREVET EUROPEEN

(43) Date de publication:
**03.02.2021 Bulletin 2021/05**

(51) Int Cl.:
***G01S 15/89*** *(2006.01)* ***G06T 7/223*** *(2017.01)*

(21) Numéro de dépôt: **20188352.7**

(22) Date de dépôt: **29.07.2020**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **29.07.2019 FR 1908627**

(71) Demandeur: **SuperSonic Imagine**
**13857 Aix-en-Provence Cedex 3 (FR)**

(72) Inventeurs:
- **SAVÉRY, David**
**37000 Tours (FR)**
- **GIRAUDAT, Elsa**
**78830 Bullion (FR)**

(74) Mandataire: **Betten & Resch**
**Patent- und Rechtsanwälte PartGmbB**
**Maximiliansplatz 14**
**80333 München (DE)**

## (54) SYSTÈME ULTRASONORE POUR DÉTECTER UN FLUX D'UN FLUIDE DANS UN MILIEU

(57) L'invention concerne un système ultrasonore pour détecter un flux d'un fluide dans un milieu, comprenant une sonde configurée pour l'insonification ultrasonore du milieu et réception d'un signal des échos, un dispositif de contrôle configuré pour construire une suite d'images basées sur le signal reçu des échos, filtrer les images par un filtre passe-haut temporel, et déterminer un déplacement local du flux entre deux images successives en maximisant la similarité entre des blocs extraits des deux images. L'invention concerne également un procédé ultrasonore pour détecter un flux d'un fluide dans un milieu.

[Fig. 2]

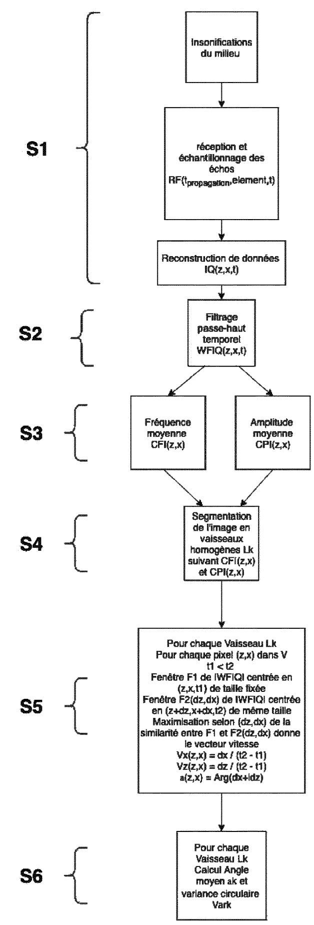


EP 3 771 927 A1

## Description

### Domaine Technique

L'invention concerne généralement l'échographie. Plus spécifiquement elle est relative à des systèmes ultrasonores, par exemple pour l'investigation ultrasonore médicale. L'invention est notamment relative à des systèmes ultrasonores pour détecter un flux d'un fluide dans un milieu, par exemple pour permettre d'estimer automatiquement la direction du flux sanguin.

### Technique antérieure

De manière connue dans l'état de la technique, un système ultrasonore peut servir pour déterminer l'angle Doppler lors d'une mesure de vélocité sanguine (ou d'un autre liquide). L'angle Doppler est l'angle entre l'axe du faisceau ultrasonore et le vecteur vitesse du sang qui s'écoule. La méthode Doppler consiste à mesurer une fréquence moyenne fDoppler à partir d'une pluralité de tirs tirés à cadence constante PRF (Pulse Repetition Frequency), et à utiliser la formule Doppler pour en déduire la vitesse Vz projetée sur l'axe du faisceau Vz = c0 ∗ fDoppler / f0 / 2 où c0 = vitesse du son et f0 = fréquence centrale de l'onde acoustique transmise. De plus, on peut calculer la vitesse absolue Va = Vz / cos(angle Doppler). Cependant, dans la méthode Doppler classique, l'échelle du spectre Doppler se détermine en une seule région (c'est-à-dire le volume d'échantillonnage) et donc dans un seul faisceau.

Dans ce cas, l'utilisateur doit identifier et sélectionner manuellement ce volume d'échantillonnage dans l'image échographique, afin d'appliquer la méthode Doppler. Par conséquent, l'angle doit être positionné à la main par l'utilisateur en mode Doppler pulsé (DP), ce qui ne permet pas d'estimation automatique de la direction du flux sanguin. Par exemple l'utilisateur détermine visuellement la direction du vaisseau par l'inspection de l'image échographique en niveaux de gris, puis indique via l'interface utilisateur la direction de l'écoulement estimée.

Par exemple, US 6,618,493 B1 propose de générer (comme le permet une insonification onde plane) K images de speckle («tavelure») à partir d'une taille de paquet (« packet size») de N avec 1 < K <N (avec K = 1 correspondant au Doppler couleur conventionnel). L'application d'un filtre passe-haut temporel de type matriciel, qui annule le tissu stationnaire, permet d'afficher à l'utilisateur les images de Color Power Imaging à une cadence typiquement augmentée d'un facteur K par rapport à l'imagerie Doppler couleur conventionnelle. Cette cadence permet de montrer un « écoulement » de speckle qui se déplace aussi latéralement.

La demande de brevet américaine US 2004/0249284 A1 décrit une méthode d'obtention du vecteur vitesse par l'utilisation de deux faisceaux ultrasonores permettant l'obtention de deux projections Doppler de la vitesse, et ainsi la détermination de l'angle Doppler dans le plan d'imagerie.

Par ailleurs, WO 2013/059659 propose d'utiliser des insonifications non focalisées (planes) pour faire l'imagerie du vecteur Doppler, d'utiliser différents angles pour évaluer les projections et combiner ces projections pour calculer le vecteur Doppler. Aussi un seul angle peut être utilisé, puis un filtre passe-haut pour calculer une carte spatio-temporelle F(p,t) où p désigne les pixels de l'image. Une méthode utilisant le gradient spatial de F(p,t) est utilisée pour calculer le vecteur vitesse en chaque point p à la date t.

Par ailleurs, US 9247927 B2 décrit un autre système ultrasonore comprenant un processeur pour visualiser le flux d'un liquide utilisant une méthode Doppler.

En outre, il a été proposé de comparer deux méthodes de speckle tracking sur des données B-mode non filtrées : la méthode dite standard qui maximise l'indice d'inter corrélation spatiale et la méthode rapide qui minimise la somme des valeurs absolues des différences (SAD), cf. par exemple :

Bohs, L. N., & Trahey, G. E. (1991). A novel method for angle independent ultrasonic imaging of blood flow and tissue motion. IEEE Transactions on Biomedical Engineering, 38(3), 280-286.

La publication suivante utilise une méthode de speckle tracking en minimisant la SAD sur une fenêtre à t et sa fenêtre décalée à t + 1/PRF du déplacement candidat ou l'intensité est calculée post wall filter (« après l'application d'un filtre passe-haut »). Les tirs sont ici focalisés et la densité de ligne est réduite par rapport au mode B pour assurer une cadence nécessaire pour éviter une trop grande décorrélation entre deux acquisitions successives :

Swillens, A., Segers, P., Torp, H., & Lovstakken, L. (2010). Two-dimensional blood velocity estimation with ultrasound: speckle tracking versus crossed-beam vector Doppler based on flow simulations in a carotid bifurcation model. IEEE transactions on ultrasonics, ferroelectrics, and frequency control, 57(2), 327-339.

### Exposé de l'invention

L'invention a pour but de pallier tout ou partie des inconvénients précités, notamment de permettre une estimation automatique de la direction d'écoulement du sang dans un ou plusieurs vaisseaux dans une image échographique.

A cet effet, l'invention propose un système ultrasonore pour détecter un flux d'un fluide dans un milieu, comprenant :

- une sonde configurée pour l'insonification ultrasonore du milieu et réception du signal des échos,
- un dispositif de contrôle configuré pour :
  - construire (en d'autres termes « reconstruire » ou « bâtir ») une suite d'images basées sur le signal reçu des échos,
  - filtrer les images par un filtre passe-haut temporel, et
  - déterminer un déplacement local du flux entre deux images successives en maximisant la similarité entre des blocs extraits des deux images.

Par conséquent, grâce à un tel dispositif de contrôle, la direction d'écoulement du sang dans un ou plusieurs vaisseaux dans une image échographique peut être estimée automatiquement. Notamment, la méthode mise en œuvre par le système permet d'afficher des spectres de vitesses dont par exemple l'angle Doppler est déterminée de manière automatique.

Par ailleurs, le système selon l'invention permet d'estimer les spectres Doppler en tout point dans le milieu examiné par le système. Cela permet l'estimation de la direction d'écoulement du sang dans plusieurs vaisseaux de manière simultanée. En comparaison, en Doppler classique comme décrit ci-dessus, l'échelle du spectre Doppler se détermine en une seule région (le volume d'échantillonnage) et l'utilisateur doit identifier et sélectionner manuellement ce volume dans l'image échographique.

Notamment le système permet l'évaluation des angles sur une pluralité d'éléments dans l'image (par exemple des vaisseaux sanguins).

Ainsi, il est possible d'évaluer l'incertitude des angles estimés, ce qui est utile pour juger de la précision et/ou de la variance de la mesure.

Avantageusement, la méthode divulguée permet d'obtenir une résolution spatiale équivalente à l'image cohérente formée par une grande ouverture et un ensemble d'angles de tirs qui est donc meilleure que celle obtenue en utilisant une sous-ouverture en réception.

En outre, par rapport aux méthodes connues dites de « speckle tracking » sur données B-mode, le système selon l'invention est avantageusement plus sensible aux flux sanguins. Les données Doppler peuvent par exemple être filtrées par un filtre de paroi sélectif du flot sanguin et suppresseur des tissus stationnaires,

De plus, la méthode mise à la disposition par le système selon l'invention est meilleure dans la mesure de la vitesse absolue que les autres méthodes Doppler connues, à multiples faisceaux, en particulier lorsque l'angle Doppler est grand (et donc l'erreur de projection est grande), puisque la mesure de vitesse absolue est d'autant plus grande que l'angle Doppler est grand, l'amplitude de vitesse étant proportionnelle à l'inverse du cosinus de l'angle Doppler. Par exemple, quand le vaisseau inspecté est horizontal sur une image échographique, et lorsque les faisceaux Doppler sont quasi-verticaux (pour des raisons de directivité des éléments de transducteurs, et donc de rapport signal-à-bruit), alors le(s) angle(s) Doppler sont grands, et l'erreur sur la vitesse absolue due à l'incertitude sur l'angle est élevée.

Le système permet de calculer de nouveaux indices diagnostiques cliniques sur par exemple le foie (par exemple évaluer l'isotropie/anisotropie de la perfusion hépatique en caractérisant une pluralité d'angles sur plusieurs vaisseaux).

Les images peuvent être filtrées par un filtre passe-haut temporel par exemple, permettant d'éliminer le signal venant du milieu stationnaire.

La similarité entre deux blocs peut être exprimée par exemple par l'inter corrélation spatiale entre les deux blocs.

Le milieu peut être stationnaire ou quasi-stationnaire. Il peut être un tissu, par exemple un organe (par exemple le foie) ou un muscle.

Les blocs peuvent comprendre au moins un premier bloc de référence dans la première des deux images et au moins un deuxième bloc de comparaison ayant la même taille dans la deuxième des deux images. Par exemple, l'algorithme de block-matching peut être appliqué afin de déterminer de manière optimale quels peuvent être les deux blocs. En effet, cet algorithme permet de localiser des blocs similaires entre deux images.

La similarité peut être maximisée en optimisant (changeant) la position du deuxième bloc, par exemple pour retrouver le premier bloc dans la deuxième image.

Le dispositif de contrôle peut être configuré pour calculer un angle moyen et/ou une vitesse moyenne du fluide basé sur le déplacement local déterminé.

L'angle peut être défini par exemple par rapport à un axe de référence de la sonde.

Le filtre passe-haut peut comprendre un filtre à réponse impulsionnelle infinie passe-haut, ou un filtre matriciel de projection orthogonale sur un sous-espace prédéfini.

La détermination du déplacement local du flux peut comprendre calculer en chaque pixel l'angle du déplacement local.

La détermination du déplacement local du flux peut comprendre une segmentation en zones homogènes des images. Chacune des zones peut correspondre à un canal, par exemple un vaisseau, comportant le fluide.

Par conséquent le dispositif de contrôle peut être configuré pour détecter un ou plusieurs canaux dans le milieu comportant le fluide en segmentant des zones homogènes dans les images.

Le dispositif de contrôle peut être configuré pour calculer un angle moyen et/ou une vitesse moyenne du fluide pour chacun des canaux.

La segmentation peut être basée sur une règle prédéfinie de décision portant sur l'amplitude et/ou la fréquence moyenne des signaux filtrés passe-haut.

La détermination du déplacement local du flux peut comprendre l'utilisation d'un algorithme de block-matching, par exemple en localisant des blocs similaires entre deux images successives. L'algorithme de block-matching peut utiliser par exemple une taille de block prédéfinie et un pas de progression de pixel prédéfini.

L'algorithme de block-matching peut être configuré pour maximiser l'inter-corrélation spatiale entre deux fenêtres de deux images filtrées successives

L'algorithme de block-matching peut utiliser une fonction croissante de l'enveloppe des signaux Doppler filtrés entre deux images successives pour déterminer un déplacement local en un ensemble de pixels (par exemple un ensemble des pixels dans un canal).

L'algorithme de block-matching peut être configuré pour utiliser l'enveloppe des signaux filtrés entre deux images successives pour déterminer un déplacement vectoriel local en un ensemble de pixels (par exemple un ensemble des pixels dans un canal).

L'algorithme de block-matching peut comprendre au moins un des algorithmes suivants :

- moyennage temporel et/ou spatial pour estimer la fonction 2D d'inter-corrélation spatiale puis la maximiser, et
- minimisation de la fonction somme des valeurs absolues des différences.

Le dispositif de contrôle peut être configuré pour calculer la variance circulaire de l'angle du flux basé sur le déplacement local déterminé sur un ensemble de pixels (par exemple un ensemble de pixels dans un canal).

L'ensemble de pixels peut être un ensemble de pixels dans un canal ou des pixels qui forme le canal sur les images.

La sonde peut être configurée pour une insonification ultra-rapide, par exemple avec une cadence d'au moins 500 tirs par seconde, plus préférablement d'au moins 3000 tirs par seconde.

La sonde peut être configurée pour une insonification à différents angles.

La sonde peut être configurée pour une insonification avec une succession de tirs d'ondes planes ultrasonores d'angles variables ou d'ondes cylindriques ultrasonores de points sources variables.

Le dispositif de contrôle peut être configuré pour construire une suite d'images démodulées en bande de base pour une cadence de répétition de tirs typique de par exemple au moins 500Hz, plus préférablement par exemple d'au moins 3000 Hz.

La sonde peut comprendre une barrette de transducteurs ultrasonores et/ou une matrice de transducteurs ultrasonores.

L'invention propose d'ailleurs un procédé ultrasonore pour détecter un flux d'un fluide dans un milieu, comprenant les étapes suivantes :

- insonification ultrasonore du milieu et réception d'un signal des échos,
- construire une suite d'images basées sur le signal reçu des échos,
- filtrer les images par un filtre passe-haut temporel, et
- déterminer un déplacement local du flux entre deux images successives en maximisant la similarité entre des blocs extraits des deux images.

Les caractéristiques et avantages de l'invention apparaitront à la lecture de la description qui va suivre, donnée uniquement à titre d'exemple non limitatif, et faite en référence aux dessins annexés.

Brève description des dessins

[Fig. 1] La figure 1 est une vue schématique d'une architecture d'un système ultrasonore selon l'invention.

[Fig. 2] La figure 2 est une vue schématique d'un procédé de traitement des données construites ultrasonores pour déterminer un flux d'un fluide dans un milieu selon l'invention.

[Fig. 3] La figure 3 illustre schématiquement un exemple d'un bloc déterminé dans un vaisseau à un temps t,

[Fig. 4] La figure 4 illustre schématiquement le calcul du déplacement local du bloc de la figure 3 à un temps t+1/PRF,

[Fig. 5] La figure 5 illustre schématiquement le calcul de la vitesse locale résultant du déplacement local du bloc de la figure 4, c'est-à-dire, la figure 5 illustre schématiquement la fonction d'intercorrélation entre les deux fenêtres illustrées sur les figures 3 et 4,

[Fig. 6] La figure 6 illustre schématiquement un exemple d'un calcul des vitesses et des angles locaux.

Description de modes de réalisation

La figure 1 est une vue schématique d'une architecture d'un système ultrasonore 1 selon l'invention. Le système 1 peut être notamment un système électronique de visualisation et il peut être par exemple configuré pour la détermination de l'angle Doppler lors d'une investigation ultrasonore médicale. Par exemple, il peut permettre d'estimer automatiquement la direction du flux sanguin.

Le système 1 comprend une sonde 3 configurée pour l'insonification ultrasonore I du milieu et réception d'un signal des échos E. La sonde peut comprendre une barrette de transducteurs ultrasonores et/ou une matrice de transducteurs ultrasonores.

En outre, le système comprend un dispositif de contrôle 2 couplé avec la sonde 3 et apte à capturer une suite d'images d'un milieu à l'aide de la sonde 3. Le dispositif 2 dans un exemple d'utilisation, construit (ou reconstruit) la suite d'images basées sur le signal reçu des échos, filtre les images par un filtre passe-haut temporel, et détermine un déplacement local du flux entre deux images successives en maximisant la similarité entre des blocs extraits des deux images. Avantageusement le dispositif peut donc estimer automatiquement la direction d'écoulement du liquide (par exemple du sang) dans un ou plusieurs vaisseaux dans une des images échographique obtenues à l'aide de la sonde. Notamment, le dispositif selon la divulgation permet d'afficher des spectres de vitesses dont par exemple l'angle Doppler est déterminé de manière automatique.

Par ailleurs, le système peut comprendre un premier écran 4 et optionnellement encore un deuxième écran 5 qui peut être tactile. L'écran 5 peut être un écran monotouche ou multitouche. Au moins un des écrans peut permettre d'afficher les spectres de vitesses.

Le système divulgué est apte à exécuter les étapes suivantes :

1. Insonification (préférablement ultrarapide) du milieu par des ondes planes ultrasonore à différents angles (typiquement par exemple 3 angles, insonification cadencée par exemple à 9000 Hz) ou par des ondes cylindriques ultrasonores à différents points-sources et réception par la sonde 3, puis,

Les étapes suivantes sont effectuées par le dispositif de contrôle 2 :

2. Construction d'une suite d'images IQ pour une cadence PRF typique de par exemple 3000 Hz;

3. Filtrage par filtre de paroi pour éliminer le signal venant des tissus stationnaires ;

4. Block matching utilisant l'enveloppe des signaux Doppler filtrés entre deux instants successifs pour déterminer un déplacement local en un ensemble de pixels (qui sont les centres des blocks). La méthode de block matching peut consister à maximiser l'inter corrélation spatiale entre deux images Doppler filtrées successives.

5. (optionnel) Calcul en chaque pixel de l'angle du déplacement local, et

6. (optionnel) Calcul sur chaque vaisseau d'un angle moyen (moyenne spatiale restreinte à une zone vaisseau), d'une vitesse moyenne, et/ou d'un vecteur de vitesse moyen.

Les étapes 2. à 6. seront décrites plus en détail dans le contexte de la figure 2.

La figure 2 est une vue schématique d'un procédé de traitement des données construites ultrasonores pour déterminer un flux d'un fluide dans un milieu selon l'invention. Le procédé peut comprendre une ou plusieurs des étapes suivantes :

Dans une étape S1 une suite d'images construites sur la base du signal des échos est obtenue. Cette étape peut comprendre les sous-étapes de :

- Insonification périodique à cadence PRF typique de par exemple 0.5 à 10 kHz du milieu par une succession d'EL tirs d'ondes planes ultrasonores d'angles variables ou d'ondes cylindriques ultrasonores d'apex (centre de courbure des fronts d'ondes) variables émises par une barrette ou une matrice de transducteurs ultrasonores.

- Réception et échantillonnage des ondes rétrodiffusées par le milieu (amplification et filtrage associé).

- Construction d'une pluralité d'images démodulées, sommation cohérente sur les angles ou sur les apex, pour obtenir une suite d'images IQ(z,x,t) complexes, où le temps lent est échantillonné à la fréquence PRFet où le nombre d'images démodulées obtenues est désigné par EL, Ensemble Length ou « taille de paquet ».

Dans une étape S2 les images sont filtrées par un filtre passe-haut (par exemple un « filtre de paroi ») temporel. Cette étape peut comprendre les sous-étapes suivantes

- Filtrage passe-haut (de « paroi ») de la suite d'images pour la suppression des échos fixes ou quasi-fixes pour obtenir une nouvelle séquence WFIQ(z,x,t). Ce filtrage peut prendre la forme d'une opération linéaire caractérisée par une matrice de transformation EL * EL pixel-dépendente de la forme :

$$[WFIQ(z,x,1) \ldots WFIQ(z,x,EL)]^T = M(z,x)\,[IQ(z,x,1) \ldots IQ(z,x,EL)]^T \quad \text{(éq. 1)}$$

M(z,x) peut être obtenue à partir de la réponse impulsionnelle d'un filtre linéaire invariant à réponse impulsionnelle finie ou infinie, ou peut être définie comme une matrice de projection sur un sous espace de $C^{EL}$ (polynômes de degrés au-dessus d'un ordre fixé, polynômes trigonométriques, etc.), ce sous-espace caractérisant les échos ultrasonores du sang s'écoulant. M(z,x) peut être variable avec le pixel (z,x) ou bien être constant dans l'image.

Dans une étape S3, une carte de vélocité moyenne et une carte d'amplitude moyenne sont calculées. Cette étape peut comprendre les sous-étapes suivantes :

- Extraction de la fréquence moyenne CFI(z,x) (color flow image) et de l'amplitude moyenne CPI(z,x) (color power image) en chaque pixel de coordonnées (z,x) à partir du vecteur [WFIQ(z,x,1) ... WFIQ(z,x,EL)]. Cette fréquence moyenne et cette amplitude moyenne peuvent se calculer par exemple à partir des relations suivantes (* désigne le complexe conjugué, arg l'argument sur [-pi,pi] d'un nombre complexe):
[Math. 1]

$$CPI = [1/(EL-1) \textstyle\sum_{t=1...EL} WFIQ(z,x,t)^* \ WFIQ(z,x,t)]^{0.5} \quad \text{(éq. 2)}$$

$$CFI = PRF * \arg(\textstyle\sum_{t=1...EL} WFIQ(z,x,t)^* \ WFIQ(z,x,t+1)\ ) / 2 / pi \quad \text{(éq. 3)}$$

Les deux cartes CFI et CPI ainsi obtenues sont ensuite utilisées pour permettre de segmenter et de labelliser les vaisseaux. Plus |CFI(z,x)| et CPI(z,x) sont grands, plus la probabilité que (z,x) soit dans un vaisseau est grande.

Dans une étape S4, un vaisseau est localisé dans la carte de vélocité moyenne. Cette étape peut comprendre :

- Segmentation en zones homogènes Lk, k = 1 ... Nk (qui peuvent se réduire en un pixel) selon les valeurs de CPI et CFI chacune des zones correspondant à un vaisseau. Une méthode de segmentation pouvant être utilisé est de diviser l'image CPI en composantes connexes dont la valeur CPI(z,x) et |CFI(z,x)| sont au-dessus de seuils prédéfinis.

Dans une étape S5, un déplacement local est déterminé pour chaque pixel dans le vaisseau, par exemple en utilisant l'algorithme de block-matching. Cette étape peut comprendre les sous-étapes suivantes :

- Pour chaque pixel (z,x) de chaque zone homogène Lk, et pour une paire de temps t1 < t2, le calcul du vecteur vitesse local v = (dz,dx)∗1/(t1-t2) est effectué en maximisant l'indice de corrélation C(dz,dx) défini par :

[Math. 2]

$$\text{Correlation Index(dz, dx)} = \frac{A_{12}(dz, dx)}{\sqrt{A_{11} * A_{22}}}$$

$$A_{11} = \sum_{window} |WFIQ(z, x, t1)|\text{^}2$$

$$A_{22} = \sum_{translated\ window} |WFIQ^*(z, x, t2)|\text{^}2$$

$$A_{12}(dz, dx) = \sum_{window} |WFIQ^*(z, x, t1)|\ |WFIQ(z + dz, x + dx, t2)|$$

où « window » désigne une fenêtre centrée en (z,x) dont la taille est prédéfinie en (z,x).

Comme indiqué dans l'exemple de la figure 5, l'indice de corrélation peut être estimé de manière plus robuste en moyennant ses valeurs à la fois spatialement et sur les temps t1 et t2, en considérant des paires t1 et t2 telle que t2-t1 soit une constante.

Comme indiqué dans l'exemple de la figure 6, si l'indice de corrélation maximisé ne dépasse pas un seuil prédéfini, alors l'estimation du déplacement local (c'est-à-dire chaque flèche indiquée dans la figure 6) peut être de mauvaise qualité et la vitesse locale [Vz(z,x) Vx(z,x)] pourrait ne pas être prise en compte dans la suite pour le calcul de certaines statistiques par exemple. Donc, le seuil prédéfini améliore avantageusement l'estimation de la vitesse et de l'angle moyens en éliminant des mesures aberrantes.

Dans une étape S6 optionnelle, pour chaque zone homogène $L_k$, un calcul d'angle moyen $\alpha_k$ peut ensuite être extrait, ainsi qu'une variance circulaire $var_k$. Ces valeurs peuvent par exemple être calculées en utilisant les formules suivantes :

$$e(z,x) = [Vx(z,x) + i\ Vz(z,x)] / |Vx(z,x) + i\ Vz(z,x)| \qquad \text{(eq. 4)}$$

$$\alpha_k = \arg(\langle e(z,x)\rangle_k) \qquad \text{(eq. 5)}$$

$$var_k = 1 - |\langle e(z,x)\rangle_k|, \qquad \text{(eq. 6)}$$

où la prise de moyenne $<>_k$ s'étant sur la zone homogène $L_k$.

Dans ce contexte, un exemple est illustré dans les figures 3 à 6.

Les figures 3 et 4 illustrent schématiquement respectivement un exemple d'un bloc déterminé dans un vaisseau à un temps t, et le calcul du déplacement local du bloc à un temps t+1/PRF. Notamment, la figure 3 illustre une image de l'amplitude d'une image Doppler d'un vaisseau incliné à un instant t1. Le rectangle blanc figure une fenêtre F1 (c'est-à-dire un bloc, par exemple déterminé par l'algorithme de bloc-matching) de taille choisie. La figure 4 illustre l'image de l'amplitude d'une image Doppler d'un vaisseau incliné à par exemple un instant t2 = t1 + 5.7 ms (ou une autre valeur prédéfinie). Le rectangle blanc figure la fenêtre F2(dz,dx) qui maximise l'intercorrélation spatiale entre F1 et une fenêtre décalée. Le déplacement évalué au centre de F1 correspond au vecteur blanc.

La figure 5 illustre schématiquement le calcul de la vitesse locale résultant du déplacement local du bloc de la figure 4, c'est-à-dire, la fonction d'intercorrélation entre les deux fenêtres illustrées les figures 3 et 4. Notamment, la figure 5 illustre l'image de l'intercorrélation spatiale (et ses lignes de niveau) entre F1 et F2(dz,dx). Elle est maximisée en par exemple dx = 3.4 mm et dz = 0.3 mm pour un indice de par exemple 0.97. C'est l'estimateur du vecteur déplacement entre t1 et t2 au centre de F1.

La figure 6 illustre schématiquement un exemple d'un calcul de vitesses et d'angles locaux. Notamment, la figure 6 illustre une image des vecteurs de vitesse calculée sur une grille de pixels d'un vaisseau incliné.

**Revendications**

1. Système ultrasonore (1) pour détecter un flux d'un fluide dans un milieu, comprenant :

   - une sonde (3) configurée pour l'insonification ultrasonore du milieu et réception d'un signal des échos,
   - un dispositif de contrôle (2) configuré pour :

     ◦ construire une suite d'images IQ(z,x,t) basées sur le signal reçu des échos,
     ◦ filtrer les images IQ(z,x,t) par un filtre passe-haut temporel, et
     ◦ déterminer un déplacement local du flux entre deux images successives en maximisant la similarité entre des blocs extraits des deux images.

2. Système de détection selon la revendication 1, dans lequel les blocs comprennent au moins un premier bloc de référence dans la première des deux images et au moins un deuxième bloc de comparaison ayant la même taille dans la deuxième des deux images, et dans lequel la similarité est maximisée en optimisant la position du deuxième bloc.

3. Système de détection selon la revendication 1 ou 2, dans lequel le dispositif de contrôle (2) est configuré pour :

   - calculer un angle moyen et/ou une vitesse moyenne du fluide basé sur le déplacement local déterminé,
   - l'angle étant défini par rapport à un axe de référence de la sonde.

4. Système selon l'une quelconque des revendications précédentes, dans lequel le filtre passe-haut comprend un filtre à réponse impulsionnelle infinie passe-haut, ou un filtre matriciel de projection orthogonale sur un sous-espace prédéfini.

5. Système selon l'une quelconque des revendications précédentes, dans lequel la détermination du déplacement local du flux comprend l'étape de calculer en chaque pixel l'angle du déplacement local.

6. Système selon l'une quelconque des revendications précédentes, dans lequel le dispositif de contrôle (2) est configuré pour détecter un ou plusieurs canaux dans le milieu comportant le fluide en segmentant des zones homogènes dans les images, et où le dispositif de contrôle est configuré pour calculer un angle moyen et/ou une vitesse moyenne du fluide pour chacun des canaux.

7. Système selon la revendication 6, dans lequel la segmentation est basée sur une règle prédéfinie de décision portant sur l'amplitude et/ou la fréquence moyenne des signaux, qui construisent la suite d'images filtrés passe-haut.

8. Système selon l'une quelconque des revendications précédentes, dans lequel la détermination du déplacement local du flux comprend l'utilisation d'un algorithme de block-matching en localisant des blocs similaires entre deux images successives.

9. Système selon la revendication précédente, dans lequel l'algorithme de block-matching est configuré pour maximiser l'inter-corrélation spatiale entre deux fenêtres de deux images filtrées successives.

10. Système selon l'une des revendications précédentes 8 ou 9, dans lequel l'algorithme de block-matching utilise une fonction croissante de l'enveloppe des signaux Doppler filtrés entre deux images successives pour déterminer un déplacement local en un ensemble de pixels.

11. Système selon l'une quelconque des revendications 8 à 10, dans lequel l'algorithme de block-matching est configuré pour utiliser l'enveloppe des signaux Doppler filtrés entre deux images successives pour déterminer un déplacement vectoriel local en un ensemble de pixels.

12. Système selon l'une quelconque des revendications précédentes 8 à 11, dans lequel l'algorithme de block-matching comprend au moins un des algorithmes suivants :

- moyennage temporel et spatial pour estimer la fonction 2D d'inter corrélation spatiale puis la maximiser, et
- minimisation de la fonction somme des valeurs absolues des différences.

**13.** Système selon l'une quelconque des revendications précédentes, dans lequel le dispositif de contrôle est configuré pour calculer la variance circulaire de l'angle du flux basé sur le déplacement local déterminé sur un ensemble de pixels.

**14.** Système selon l'une quelconque des revendications 10 à 13, dans lequel l'ensemble de pixels est un ensemble de pixels dans un canal.

**15.** Système de détection selon l'une quelconque des revendications précédentes, dans lequel la sonde (3) est configurée pour :

- une insonification ultra-rapide avec une cadence d'au moins 500 tirs par seconde, et/ou
- une insonification à différents angles, et/ou
- une insonification avec une succession de tirs d'ondes planes ultrasonores d'angles variables ou d'ondes cylindriques ultrasonores de sources variables, et/ou
- le dispositif de contrôle est configuré pour construire une suite d'images démodulées en bande de base pour une cadence de répétition de tirs de au moins 500Hz.

**16.** Procédé ultrasonore pour détecter un flux d'un fluide dans un milieu, comprenant les étapes :

- d'insonification ultrasonore du milieu et réception d'un signal des échos,
- construction d'une suite d'images basées sur le signal reçu des échos,
- filtrage des images par un filtre passe-haut temporel, et
- détermination d'un déplacement local du flux entre deux images successives en maximisant la similarité entre des blocs extraits des deux images.

[Fig. 1]

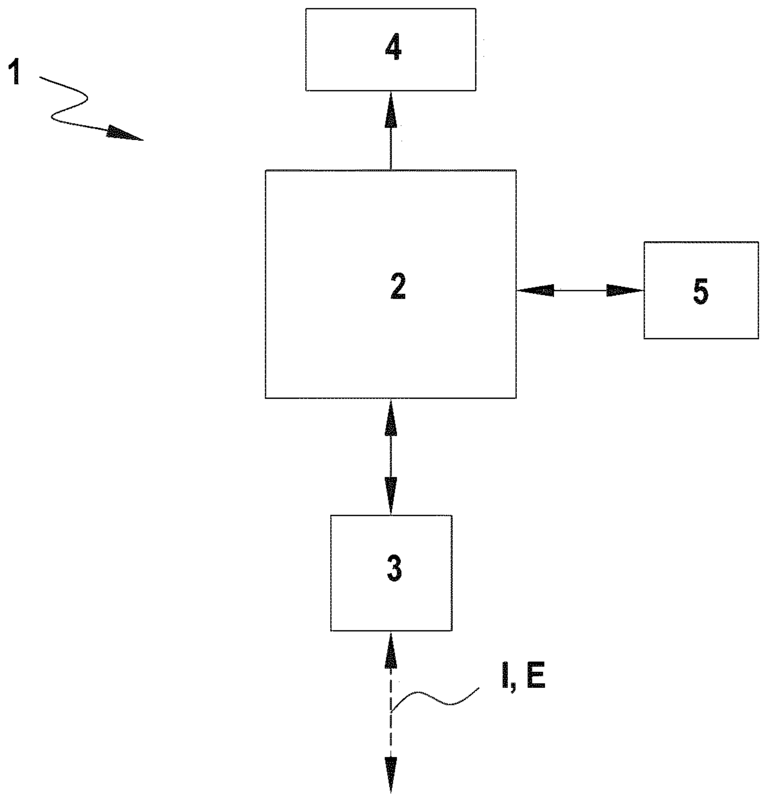

[Fig. 2]

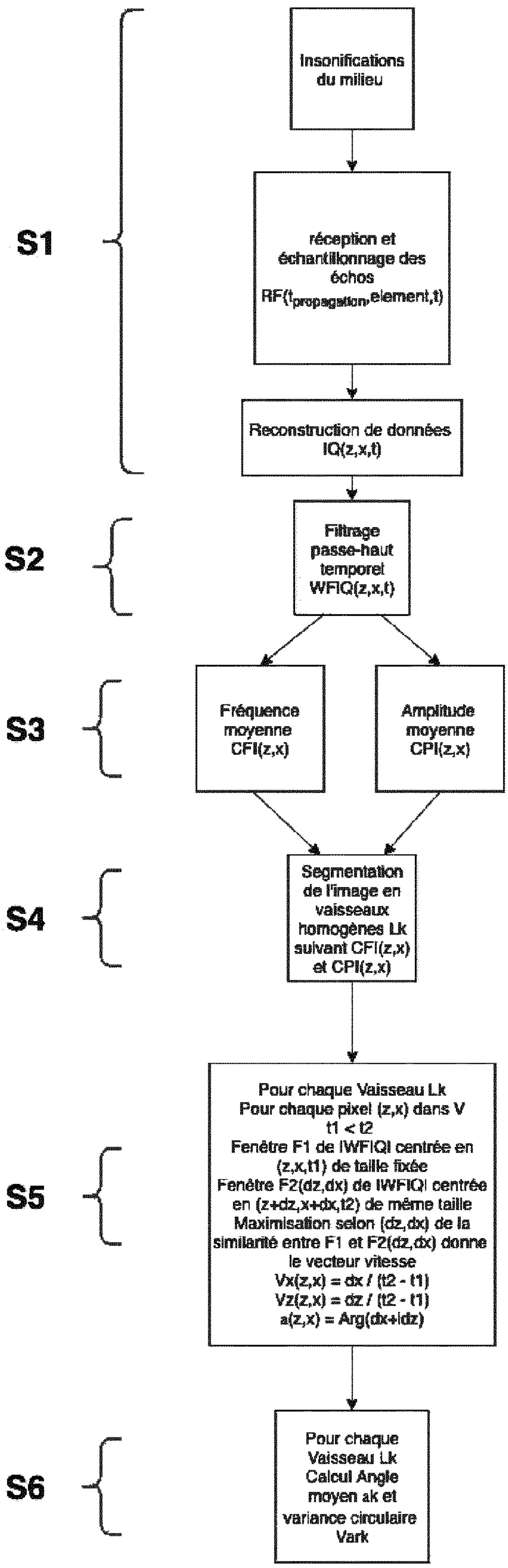
S1
Insonifications du milieu
réception et échantillonnage des échos
RF(tpropagation,element,t)
Reconstruction de données IQ(z,x,t)
S2
Filtrage passe-haut temporel WFIQ(z,x,t)
S3
Fréquence moyenne CFI(z,x)
Amplitude moyenne CPI(z,x)
S4
Segmentation de l'image en vaisseaux homogènes Lk suivant CFI(z,x) et CPI(z,x)
S5
Pour chaque Vaisseau Lk
Pour chaque pixel (z,x) dans V
t1 < t2
Fenêtre F1 de IWFIQI centrée en (z,x,t1) de taille fixée
Fenêtre F2(dz,dx) de IWFIQI centrée en (z+dz,x+dx,t2) de même taille
Maximisation selon (dz,dx) de la similarité entre F1 et F2(dz,dx) donne le vecteur vitesse
Vx(z,x) = dx / (t2 - t1)
Vz(z,x) = dz / (t2 - t1)
a(z,x) = Arg(dx+idz)
S6
Pour chaque Vaisseau Lk
Calcul Angle moyen ak et variance circulaire Vark

[Fig. 3]

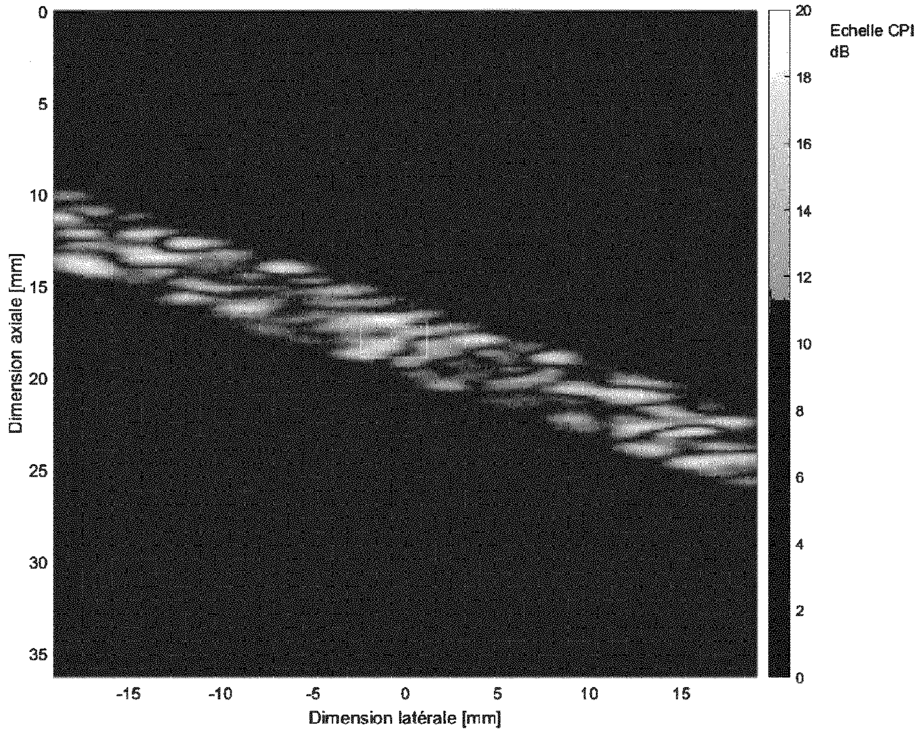
Echelle CPI
dB
20
18
16
14
12
10
8
6
4
2
0
Dimension axiale [mm]
0
5
10
15
20
25
30
35
-15
-10
-5
0
5
10
15
Dimension latérale [mm]

[Fig. 4]

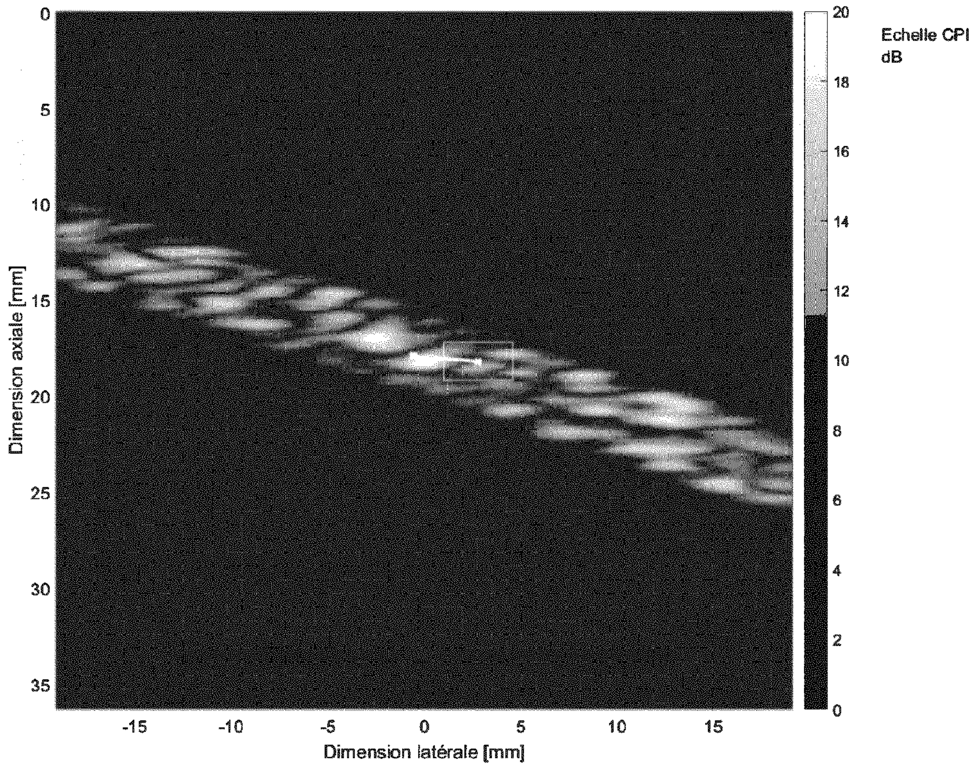
Echelle CPI
dB
20
18
16
14
12
10
8
6
4
2
0
Dimension axiale [mm]
0
5
10
15
20
25
30
35
-15
-10
-5
0
5
10
15
Dimension latérale [mm]

[Fig. 5]

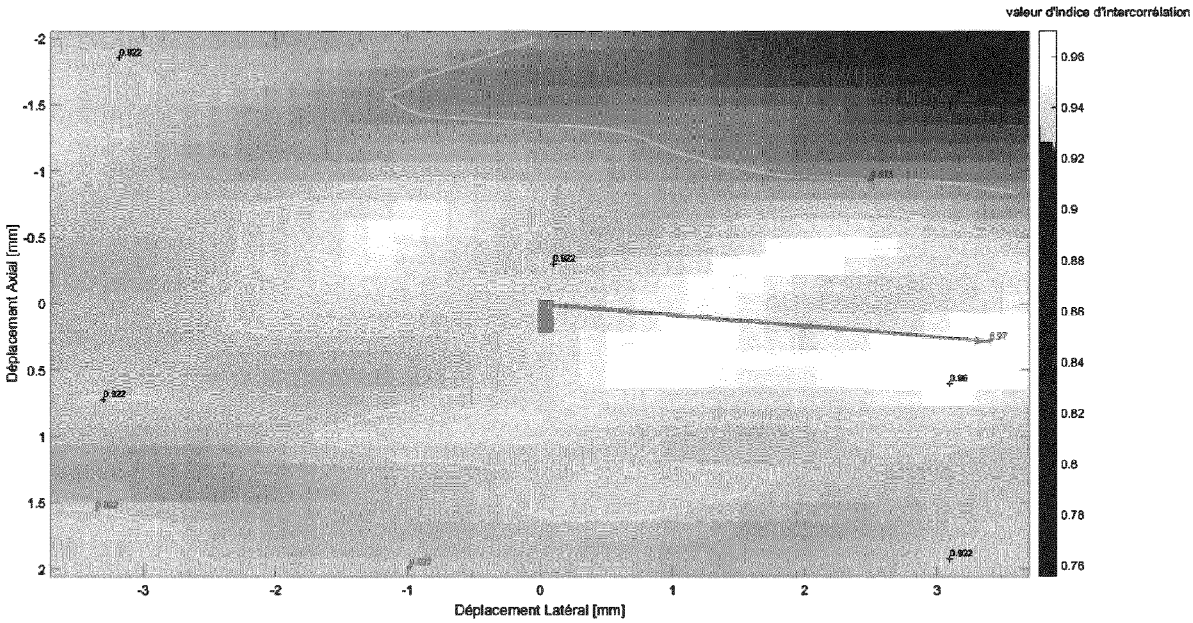
valeur d'indice d'intercorrélation
Déplacement Axial [mm]
Déplacement Latéral [mm]

[Fig. 6]

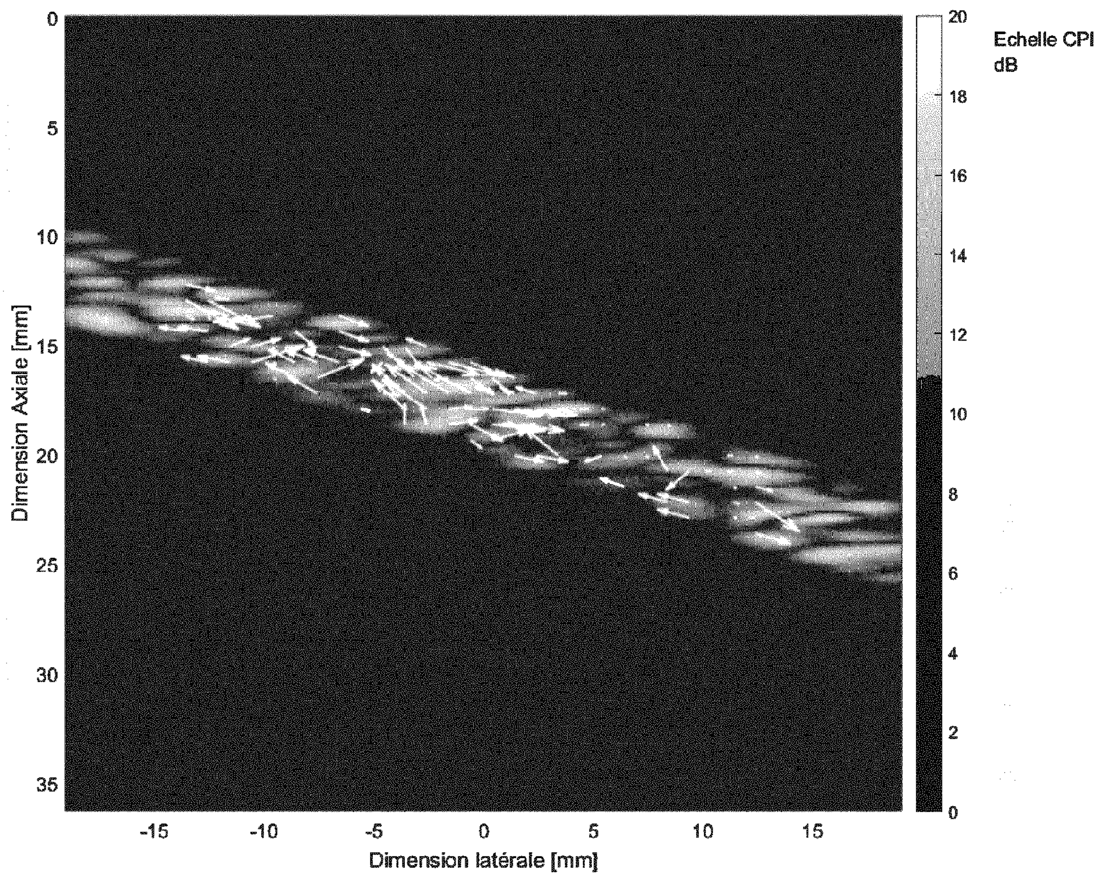
Echelle CPI
dB
Dimension Axiale [mm]
Dimension latérale [mm]

Europäisches Patentamt
European Patent Office
Office européen des brevets

# RAPPORT DE RECHERCHE EUROPEENNE

Numéro de la demande
EP 20 18 8352

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | PERROT VINCENT ET AL: "Back to Basics in Ultrasound Velocimetry: Tracking Speckles by Using a Standard Piv Algorithm", 2018 IEEE INTERNATIONAL ULTRASONICS SYMPOSIUM (IUS), IEEE, 22 octobre 2018 (2018-10-22), pages 206-212, XP033479862, DOI: 10.1109/ULTSYM.2018.8579665 * le document en entier * ----- | 1-16 | INV. G01S15/89 G06T7/223 |
| A | WO 2018/208942 A1 (UNIV NORTH CAROLINA CHAPEL HILL [US]) 15 novembre 2018 (2018-11-15) * le document en entier * ----- | 1-16 | |

DOMAINES TECHNIQUES RECHERCHES (IPC)
G01S
G06T

1 Le présent rapport a été établi pour toutes les revendications

| Lieu de la recherche | Date d'achèvement de la recherche | Examinateur |
|---|---|---|
| Munich | 23 novembre 2020 | Zaneboni, Thomas |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 20 18 8352

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-11-2020

| Document brevet cité au rapport de recherche | Date de publication | Membre(s) de la famille de brevet(s) | Date de publication |
|---|---|---|---|
| WO 2018208942 A1 | 15-11-2018 | US 2020187910 A1 | 18-06-2020 |
| | | WO 2018208942 A1 | 15-11-2018 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6618493 B1
- US 20040249284 A1
- WO 2013059659 A
- US 9247927 B2


### Littérature non-brevet citée dans la description


- **BOHS, L. N. ; TRAHEY, G. E.** A novel method for angle independent ultrasonic imaging of blood flow and tissue motion. *IEEE Transactions on Biomedical Engineering,* 1991, vol. 38 (3), 280-286
- **SWILLENS, A. ; SEGERS, P. ; TORP, H. ; LOVSTAKKEN, L.** Two-dimensional blood velocity estimation with ultrasound: speckle tracking versus crossed-beam vector Doppler based on flow simulations in a carotid bifurcation model. *IEEE transactions on ultrasonics, ferroelectrics, and frequency control,* 2010, vol. 57 (2), 327-339